# EUROPEAN PATENT APPLICATION

(11) **EP 4 349 848 A1**
(43) Date of publication of application: **10.04.2024**
(21) Application number: 22815945.5
(22) Date of filing: 25.05.2022
(51) Int. Cl.: C07K 1/08

(54) **PEPTIDE COMPOUND PRODUCTION METHOD AND AMIDATION AGENT**

(30) Priority: 31.05.2021 JP 2021091438
(71) Applicant: Chubu University Educational Foundation, Kasugai-shi Aichi 487-8501 (JP)
(72) Inventor: YAMAMOTO, Hisashi, Kasugai-shi, Aichi 487-8501 (JP); HATTORI, Tomohiro, Kasugai-shi, Aichi 487-8501 (JP)
(74) Representative: Lavoix
(86) International application number: PCT/JP2022/021434
(87) International publication number: WO 2022/255195

(57) **Abstract**

The present invention provides a method for inexpensively and efficiently synthesizing polypeptide compounds comprising various amino acids. The method involves (i) inducing an amide formation reaction between the carboxyl group on the right side in the formula of the amino acid or peptide compound represented by formula (R1) and the amino group on the left side in the formula of the amino acid ester or peptide ester compound represented by formula (R2) in the presence of an aluminum compound represented by formula (A) to obtain a peptide compound represented by formula (P1) (the definitions of the reference signs in the formula are as presented in the description).

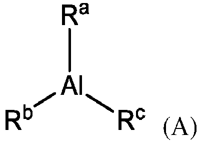

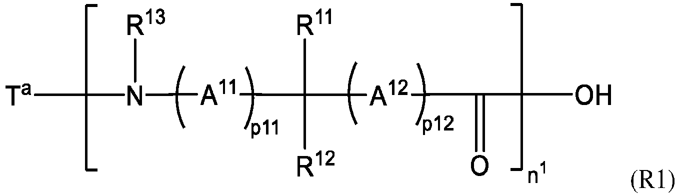

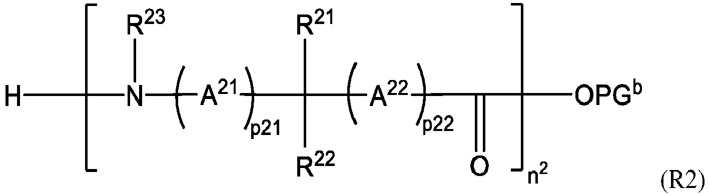

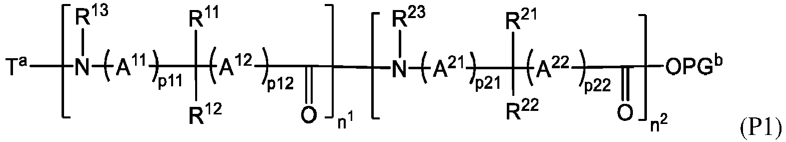

## Description

### FIELD

The present invention relates to a novel production method of a peptide compound and an amide forming reaction agent for use in the production method.

### BACKGROUND

Conventionally, amide compounds represented by peptides have been used in a wide variety of fields, including pharmaceuticals, cosmetics, and functional foods. Development of synthetic methods thereof has been diligently pursued as an important research goal in synthetic chemistry (Non-Patent Literature 1 to 3). However, there are few truly effective catalysts or reaction agents other than carboxylic acid activators for the amidation reaction, which is the most important reaction in peptide synthesis. Therefore, it is unavoidable to use a reaction mode that forms by-products, and thus, peptide synthesis, which involves repeating multi-stage reactions, is extremely inefficient from the viewpoint of atom economy (atomic yield). The amount of by-products is large, and there are few effective purification means. As a result, the cost of disposal of by-products and purification constitutes most of the necessary costs for peptide synthesis, and is the largest obstacle to development in this field.

In peptide synthesis, which uses amino acids or derivatives thereof as starting materials, it is desirable for the amidation reaction to proceed with high stereoselectivity. Enzyme reactions in the body are examples of highly stereoselective amidation reactions. For example, in the body, peptides are synthesized with extremely high stereoselectivity through sophisticated use of enzymes and hydrogen bonds. However, enzyme reactions are not suitable for mass production, requiring enormous financial and time costs when applied to synthetic chemistry.

In synthetic chemistry, amidation reactions using catalysts have been examined, but in conventional means, the amide bond is formed primarily through the method of activating carboxylic acid, such that racemization occurs quickly, whereby synthesizing a peptide with high stereoselectivity and efficiency is difficult.

According to conventional methods, it is very difficult to link an additional amino acid or derivative to a peptide comprising a plurality of amino acids or derivatives thereof (chemical ligation) or link two or more peptides via amide bonds. As an amidation method for ligation to such peptides, there are known a method for ligation by using an amino acid having a sulfur atom to utilize the high reactivity of the sulfur atom (Non-Patent Literature 4) and a method for ligation by synthesizing an amino acid hydroxyamine to utilize the high reactivity of the hydroxyamine (Non-Patent Literature 5). However, in the former method, it is difficult to synthesize amino acids having a sulfur atom, and in the latter method, hydroxyamine synthesis involving several steps is necessary. Both methods are time-consuming and costly and have a disadvantage in efficiency.

The present inventors have developed, as techniques for synthesizing an amide compound in a highly chemoselective manner: a method of amidating a carboxylic acid/ester compound having a hydroxy group at the β-position in the presence of a specific metal catalyst (Patent Literature 1); a method of using a hydroxyamino/imino compound as an amino acid precursor and amidating it in the presence of a specific metal catalyst, and then reducing them in the presence of a specific metal catalyst (Patent Literature 2); and a method of amidating a carboxylic acid/ester compound in the presence of specific metal catalyst (Patent Literature 3). The present inventors have also developed a technique for highly efficient and selective synthesis of peptides consisting of various amino acid residues by amide reaction of the carboxyl group of an N-terminal protected amino acid/peptide and the amino group of a C-terminal protected amino acid/peptide in the presence of a specific silylating agent and an optionally used Lewis acid catalyst, followed by deprotection (Patent Literature 4). The present inventors have further developed a method of synthesizing a peptide composed of various amino acid residues with a high efficiency and in a highly selective manner, by causing an amide reaction a carboxyl group of an amino acid or peptide whose N-terminal is either protected or unprotected and an amino group of an amino acid or peptide whose C-terminal is either protected or unprotected in the presence of a specific silylating agent, followed by deprotection (Patent Literatures 5 and 6), and a method for causing an amidation reaction using a Bronsted acid as a catalyst (Patent Literature 7).

In recent years, attempts have been made to synthesize peptides using unprotected amino acids (Patent Literature 6 to Patent Literature 8), but none of them were satisfactory in terms of the types of amino acids available or reaction efficiency.

### CITATION LIST

### Patent Literature

[Patent Literature 1] WO2017/204144 A
[Patent Literature 2] WO2018/199146 A
[Patent Literature 3] WO2018/199147 A
[Patent Literature 4] WO2019/208731 A
[Patent Literature 5] WO2021/085635 A
[Patent Literature 6] WO2021/085636 A
[Patent Literature 7] WO2021/149814 A

### Non-Patent Literature

[Non-Patent Literature 1] Chem. Rev., 2011, Vol.111, p.6557-6602
[Non-Patent Literature 2] Org. Process Res. Dev., 2016, Vol.20, No.2, p.140-177
[Non-Patent Literature 3] Chem. Rev., 2016, Vol.116, p.12029-12122
[Non-Patent Literature 4] Science, 1992, Vol.256, p.221-225
[Non-Patent Literature 5] Angew. Chem. Int. Ed., 2006, Vol.45, p.1248-1252
[Non-Patent Literature 6] Chem. Eur. J. 2019, Vol.25, p.15091
[Non-Patent Literature 7] Org. Lett. 2020, Vol.22, p.8039
[Non-Patent Literature 8] Chem. Eur. J. 2018, Vol.24, p.7033
[Non-Patent Literature 9] Synlett, 2011, p.2072-2074

### SUMMARY

### Problem to be Solved by the Invention

Against this background, there is a need for an inexpensive and efficient method for synthesizing polypeptides composed of various amino acids. The present invention has been made in view of this problem.

### Means for Solving the Problem

As a result of intensive investigations, the present inventors have found that aluminum compounds having specific structures have the effect of causing an amide-forming reaction between terminal carboxyl groups and terminal amino groups of various amino acids and peptides. The present inventors have also found that the use of such aluminum compounds as amide forming reaction agents makes it possible to cause amidation reactions between terminal carboxyl groups and terminal amino groups of various amino acids and peptides as substrates in a highly stereoselective and/or efficient manner, thereby enabling inexpensive and efficient synthesis of peptide compounds composed of various amino acids. The present inventors have further found that the use of such aluminum compounds as amide forming reaction agents makes it possible to cause amidation reactions between unprotected N-terminals of amino acid esters or peptide esters as electrophilic amino acids and terminal amino groups of amino acid or peptides as nucleophiles. Based on these findings, the present inventors have completed the present invention.

There have been reports of the use of trimethylaluminum in the amidation reaction between carboxylic acid compounds and amino compounds (Non-Patent Literature 9). However, it has not been known to the art so far, and has been newly found by the present inventors, that aluminum compounds with specific structures such as trimethylaluminum can be used as reaction agents for peptide synthesis via amidation between amino acids and/or peptides. The findings by the present inventors are surprising also because amidation reactions using amino acid esters or peptide esters as electrophilic species have rarely been reported so far.

Thus, the present invention provides the following aspects.
[Aspect 1]
   A method for producing a polypeptide compound, comprising the step of (i) causing an amide forming reaction between the carboxyl group on the right side of an amino acid or peptide compound represented by formula (R1) and the amino group on the left side of represented by formula (R2) amino acid ester or peptide ester compound in the presence of an aluminum compound represented by formula (A), to produce a peptide compound represented by formula (P1). 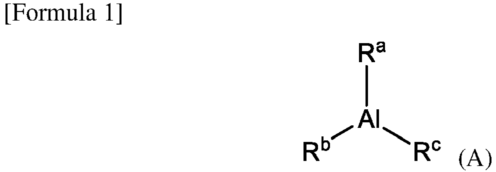
   In formula (A),
   R^{a}, R^{b}, and R^{c} each represent, independently of each other, a hydrogen atom, halogen atom, hydroxyl group, carboxyl group, nitro group, cyano group, or thiol group, or a monovalent aliphatic hydrocarbon group, aromatic hydrocarbon group, heterocyclic group, aliphatic hydrocarbon oxy group, aromatic hydrocarbon oxy group, heterocyclic group-substituted oxy group, or metalloxy group that may have one or more substituents. 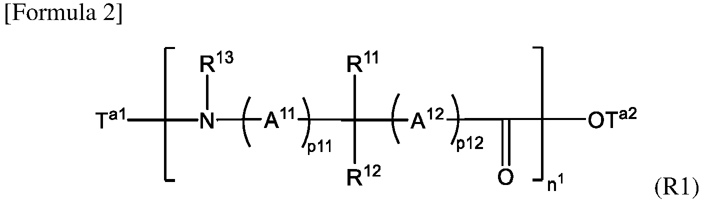
   In formula (R1),
   T^{a1} and T^{a2} each represent, independently of each other, a hydrogen atom or monovalent substituent,
   R¹¹ and R¹² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
   R¹³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or R¹¹ and R¹³ may be bound to each other to form, together with the carbon atom to which R¹¹ binds and the nitrogen atom to which R¹³ binds, a heterocyclic group that may have one or more substituents,
   A¹¹ and A¹² each represent, independently of each other, a divalent aliphatic hydrocarbon group that may have one or more substituents having 1 to 3 carbon atoms,
   p11 and p12 each represent, independently of each other, 0 or 1, and
   n¹ represents an integer of equal to or greater than 1 corresponding to the number of the structure units parenthesized with [ ], provided that when n¹ is equal to or greater than 2, then the two or more structure units in [ ] may be either identical to each other or different from each other. 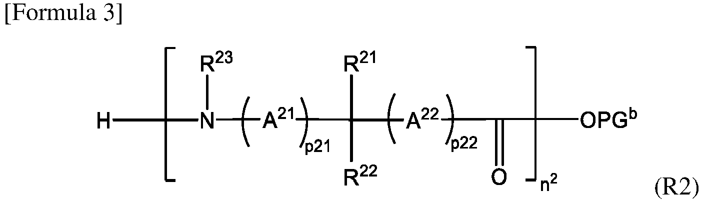
   In formula (R2),
   PG^{b} represents a protective group for carboxyl groups,
   R²¹and R²² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
   R²³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
   R²¹ and R²³ may be bound to each other to form, together with the carbon atom to which R²¹ binds and the nitrogen atom to which R²³ binds, a heterocyclic group that may have one or more substituents,
   A²¹ and A²² each represent, independently of each other, a divalent aliphatic hydrocarbon group that may have one or more substituents having 1 to 3 carbon atoms,
   p21 and p22 each represent, independently of each other, 0 or 1, and
   n² represents an integer of equal to or greater than 1 corresponding to the number of the structure units parenthesized with [ ], provided that when n² is equal to or greater than 2, then the two or more structure units in [ ] may be either identical to each other or different from each other. 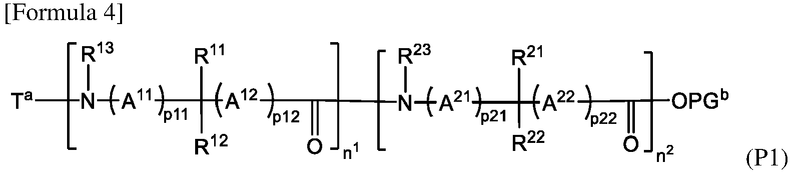
   In formula (P1),
   T^{a}, R¹¹, R¹², R¹³, A¹¹, A¹², p11, p12, and n¹ each represent the same definitions as those of the same symbols in general formula (R1) above, and
   PG^{b}, R²¹, R²², R²³, A²¹, A²², p21, p22, and n² each represent the same definitions as those of the same symbols in general formula (R2) above.
[Aspect 2] The method according to Aspect 1, further comprising, after step (i), the step of (ii) causing an amide forming reaction between the carboxyl group on the right side of an amino acid or peptide compound represented by formula (R3) and the amino group on the left side of the peptide ester compound represented by formula (P1) to thereby produce a peptide compound represented by formula (P2). 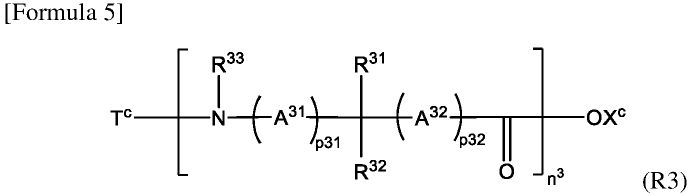 In formula (R3),
   T^{c} represents a hydrogen atom or monovalent substituent,
   X^{c} represents a halogen atom, hydroxyl group, or a monovalent aliphatic hydrocarbon group or aromatic hydrocarbon group that may have one or more substituents,
   R³¹and R³² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
   R³³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
   R³¹ and R³³ may be bound to each other to form, together with the carbon atom to which R³¹ binds and the nitrogen atom to which R³³ binds, a heterocyclic group that may have one or more substituents,
   A³¹ and A³² each represent, independently of each other, a divalent aliphatic hydrocarbon group that may have one or more substituents having 1 to 3 carbon atoms,
   p31 and p32 each represent, independently of each other, 0 or 1, and
   n³ represents an integer of equal to or greater than 1 corresponding to the number of the structure units parenthesized with [ ], provided that when n³ is equal to or greater than 2, then the two or more structure units in [ ] may be either identical to each other or different from each other. 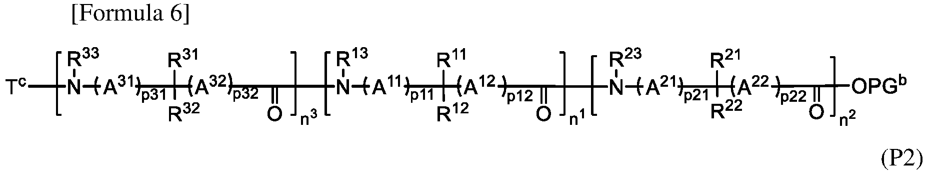
   In formula (P2),
   R¹¹, R¹², R¹³, A¹¹, A¹², p11, p12, and n¹ each represent the same definitions as those of the same symbols in general formula (R1) above,
   PG^{b}, R²¹, R²², R²³, A²¹, A²², p21, p22, and n² each represent the same definitions as those of the same symbols in general formula (R2) above, and
   T^{c}, R³¹, R³², R³³, A³¹, A³², p31, p32, and n³ each represent the same definitions as those of the same symbols in general formula (R3) above.
[Aspect 3] The method according to Aspect 1 or 2, wherein a silane compound is added to the reaction system.
[Aspect 4] The method according to any one of Aspects 1 to 3, wherein the reaction is carried out as a batch reaction or a flow reaction.
[Aspect 5] An amidation reaction agent for use in the method according to any one of Aspects 1 to 4, comprising an aluminum compound represented by formula (A). 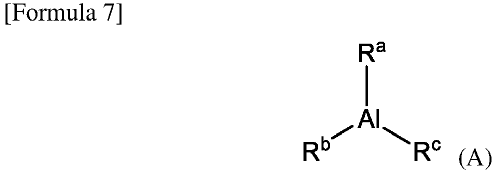
   In formula (A),
   R^{a}, R^{b}, and R^{c} each represent, independently of each other, a hydrogen atom, halogen atom, hydroxyl group, carboxyl group, nitro group, cyano group, or thiol group, or a monovalent aliphatic hydrocarbon group, aromatic hydrocarbon group, heterocyclic group, aliphatic hydrocarbon oxy group, aromatic hydrocarbon oxy group, or heterocyclic oxy group that may have one or more substituents.

### Effects of the Invention

According to the method of the present invention, the use of aluminum compounds having specific structures as an amide forming reaction agent makes it possible to cause amidation reactions between terminal carboxyl groups and terminal amino groups of various amino acids and peptides as substrates in a highly stereoselective and/or efficient manner, thereby enabling inexpensive and efficient synthesis of peptide compounds composed of various amino acids. The use of such aluminum compounds as amide forming reaction agents makes it possible to cause amidation reactions between unprotected N-terminals of amino acid esters or peptide esters as electrophilic amino acids and terminal amino groups of amino acid or peptides as nucleophiles.

### EMBODIMENTS

The present invention is described hereinafter in detail with reference to specific embodiments thereof. However, the present invention is not limited to the following embodiments and can be carried out in any embodiment that does not deviate from the gist according to the present invention.

All patent publications, patent application publications, and non-patent documents cited in this disclosure are incorporated herein by reference in their entirety for all purposes.

### [I. Definitions of Terms]

The term "amino acid" herein refers to a compound having a carboxyl group and an amino group. Unless otherwise specified, the type of an amino acid is not particularly limited. For example, from the viewpoint of optical isomerism, an amino acid may be in the D-form, in the L-form, or in a racemic form. From the viewpoint of the relative positions of the carboxyl group and the amino group, an amino acid may be any of an α-amino acid, β-amino acid, γ-amino acid, δ-amino acid, or ε-amino acid. Examples of amino acids include, but are not limited to, natural amino acids that make up proteins. Examples include valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, and serine.

The term "peptide" herein refers to a compound comprising a plurality of amino acids linked together via peptide bonds. Unless otherwise specified, the plurality of amino acid units constituting a peptide may be the same type of amino acid unit or may consist of two or more types of amino acid units. The number of amino acids constituting a peptide is not restricted as long as it is two or more. Examples include 2 (also called "dipeptide"), 3 (also called "tripeptide"), 4 (also called "tetrapeptide"), 5 (also called "pentapeptide"), 6, 7, 8, 9, 10, 15, 20, 30, 40, 50, 100, or more. The term "polypeptide" may also be used to refer to tripeptides and longer peptides.

The term "amino group" herein refers to a functional group represented by any formula of -NH₂, -NRH, and -NRR' (where R and R' each represent a substituent) obtained by removing hydrogen from ammonia, a primary amine, and a secondary amine, respectively.

Unless otherwise specified, a hydrocarbon group herein may be either aliphatic or aromatic. An aliphatic hydrocarbon group may be in the form of either a chain or a ring. A chain hydrocarbon group may be linear or branched. A cyclic hydrocarbon group may be monocyclic, bridged cyclic, or spirocyclic. The hydrocarbon group may be saturated or unsaturated. In other words, one, two, or more carbon-carbon double and/or triple bonds may be included. Specifically, "hydrocarbon group" represents a concept including an alkyl group, alkenyl group, alkynyl group, cycloalkyl group, cycloalkenyl group, cycloalkynyl group, aryl group, etc. Unless otherwise specified, one, two, or more hydrogen atoms of the hydrocarbon group may be replaced with any substituents and one, two, or more carbon atoms in the hydrocarbon group may be replaced with any heteroatoms corresponding to the valence thereof.

The term "hydrocarbon oxy group" herein refers to a group comprising an oxy group (-O-) linked via one bond thereof to the hydrocarbon group as defined above.

The term "hydrocarbon carbonyl group" herein refers to a group comprising a carbonyl group (-C(=O)-) linked via one bond thereof to the hydrocarbon group as defined above.

The term "hydrocarbon sulfonyl group" herein refers to a group comprising a sulfonyl group (-S(=O)₂-) linked via one bond thereof to the hydrocarbon group as defined above.

A heterocyclic group may be saturated or unsaturated. In other words, it may contain one, two, or more carbon-carbon double and/or triple bonds. A heterocyclic group may be monocyclic, bridged cyclic, or spirocyclic. The heteroatom included in the constituent atoms of the heterocyclic group is not particularly limited, examples thereof including nitrogen, oxygen, sulfur, phosphorus, and silicon.

The term "heterocyclic oxy group" herein refers to a group comprising an oxy group (-O-) linked via one bond thereof to the heterocyclic group as defined above.

The term "heterocyclic carbonyl group" herein refers to a group comprising a carbonyl group (-C(=O)-) linked via one bond thereof to the heterocyclic group as defined above.

The term "heterocyclic sulfonyl group" herein refers to a group comprising a sulfonyl group (-S(=O)₂-) linked via one bond thereof to the heterocyclic group as defined above.

The term "metalloxy group" (that may have one or more substituents) herein refers to a group represented by formula (R)ₙ-M-O-, where M refers to a metal element, R refers to a substituent, n refers to an integer of 0 or more but 8 or less corresponding to the coordination number of the metal element M.

Unless otherwise specified, the term "substituent" herein refers, independently of each other, to any substituent which is not particularly limited so long as the amidation step of the production method according to the present invention proceeds. Examples include, but are not limited to, a halogen atom, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyloxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyloxy group, heterocyclic oxy group, heterocyclic carbonyl group, heterocyclic oxycarbonyl group, heterocyclic carbonyloxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyloxy group. The term "substituents" also may include functional groups obtained by substituting any of the aforementioned functional groups with any of the aforementioned functional groups as long as the valence and physical properties thereof permit. When a functional group has one or more substituents, the number of the substituents is not particularly limited as long as the valence and physical properties thereof permit. When the functional group has two or more substituents, they may be identical to each other or different from each other.

The main abbreviations used herein are listed in Table 1 below.

**[Table 1]**

| Abbreviation | Definition |
|---|---|
| Ac | acetyl |
| acac | acetyl acetonate |
| Alloc | allyoxycarbonyl |
| Bn or Bzl | benzyl |
| Boc | tert-butoxycarbonyl |
| Bu | butyl |
| Bz | benzoyl |
| Cbz | benzyloxycarbonyl |
| Cp | cyclopentadienyl |
| 2,4-DNP | 2,4-dinitrophenyl |
| Et | ethyl |
| Fmoc | 9-fluorenylmethyloxycarbonyl |
| HOBt | 1-hydroxybenzotriazole |
| i-Pr | isopropyl |
| MABR | methylaluminum bis(4-bromo-2,6-di-tert-butylphenoxyde) |
| Me | methyl |
| Ms | mesyl |
| Ns | 2 or 4-nitrobenzenesulfonyl |
| Phth | phthaloyl |
| PMB | paramethoxybenzyl |
| PMPCO | paramethoxybenzoyl |
| Pr | propyl |
| TBAF | tetrabutylammonium fluoride |
| TBDPS | tert-butyl diphenylsilyl |
| TBS | tritert-butylsilyl |
| t-Bu | tert-butyl |
| TES | triethylsilyl |
| Tf | trifluoromethane sulfonyl |
| THF | tetrahydrofuran |
| TIPS | triisopropylsilyl |
| TMS | trimethylsilyl |
| TMS-IM | trimethylsilyl imidazole |
| TMS-OTf | trimethylsilyl trifluoromethanesulfonate |
| Troc | 2,2,2-trichloroethoxy carbonyl |
| Trt | trityl |
| Ts | toluenesulfonyl |
| wscHCl | 1-ethyl-3-(3-dimethylaminopropyl)carbodiimide hydrochloride |

Amino acids and residues thereof may herein be represented by three-letter abbreviations well known to a person skilled in the art. The three-letter abbreviations of major amino acids are shown in the following table.

**[Table 2]**

| Abbreviation | Definition |
|---|---|
| Ala | Alanine |
| Arg | Arginine |
| Asn | Asparagine |
| Asp | Aspartic acid |
| Cvs | Cysteine |
| Gln | Glutamine |
| Glu | Glutamic acid |
| Glv | Glycine |
| His | Histidine |
| Ile | Isoleucine |
| Leu | Leucine |
| Lys | Lysine |
| Met | Methionine |
| Phe | Phenylalanine |
| Phg | Phenylglycine |
| Pro | Proline |
| Ser | Serine |
| Thr | Threonine |
| Trp | Tryptophan |
| Tyr | Tyrosine |
| Val | Valine |

β-homoamino acids and residues thereof may herein be represented by "Ho" followed by three-letter abbreviations of corresponding β-amino acids.

### [II. Amidation Reaction Agent]

An embodiment according to the present invention relates to an amidation reaction agent containing an aluminum compound represented by formula (A) (hereinafter also referred to as the "amidation reaction agent of the present invention").

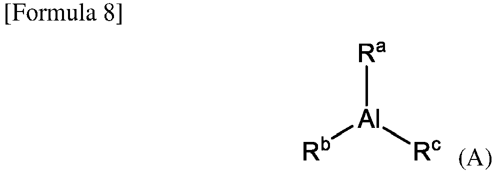

In formula (A), R^{a}, R^{b}, and R^{c} each represent, independently of each other, a hydrogen atom, halogen atom, hydroxyl group, carboxyl group, nitro group, cyano group, or thiol group, or a monovalent aliphatic hydrocarbon group, aromatic hydrocarbon group, heterocyclic group, aliphatic hydrocarbon oxy group, aromatic hydrocarbon oxy group, heterocyclic group-substituted oxy group, or metalloxy group that may have one or more substituents. If these groups have one or more substituents, they may be selected arbitrarily from those detailed earlier. The number of substituents is not restricted, but may be 5, 4, 3, 2, 1, or 0.

In formula (A), when each of R^{a}, R^{b}, and/or R^{c} is a monovalent aliphatic hydrocarbon group, aromatic hydrocarbon group, or heterocyclic group that may have one or more substituents, a linking group may intervene between the aliphatic hydrocarbon group, aromatic hydrocarbon group, or heterocyclic group and the carbon atom to which it binds. The linking group may be, independently of each other, selected from, although is not limited to, the structures listed below (where, in the chemical formulae below, A represents, independently of each other, a monovalent aliphatic hydrocarbon group, aromatic hydrocarbon group, or heterocyclic group that may have one or more substituents. When two A's are present in the same group, they may be identical to each other or different from each other.).

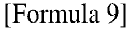

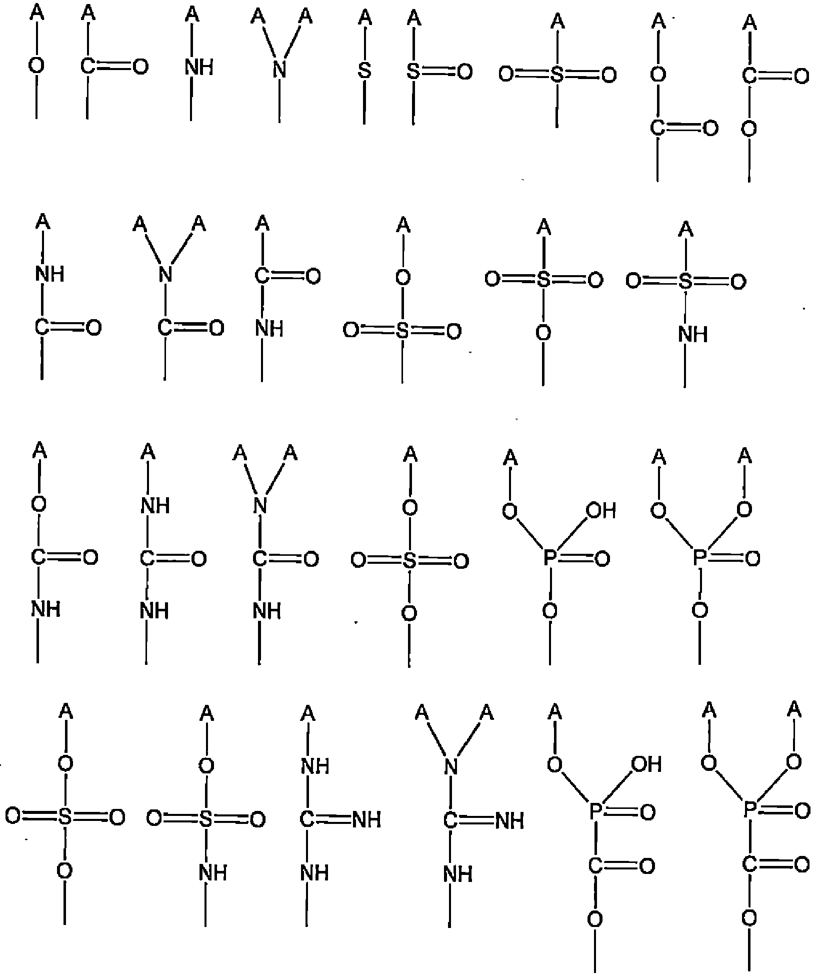

In formula (A), when each of R^{a}, R^{b}, and/or R^{c} is an aliphatic hydrocarbon group (that may have one or more substituents), the number of carbon atoms in the aliphatic hydrocarbon group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the aliphatic hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

In formula (A), when each of R^{a}, R^{b}, and/or R^{c} is an aromatic hydrocarbon group (that may have one or more substituents), the number of carbon atoms in the aromatic hydrocarbon group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the aromatic hydrocarbon group, but may be 4 or more, for example 5 or more, or 6 or more. Specific examples of the number of atoms include 4, 5, 6, 7, 8, 9, 10, 11, 12, 13, 14, 15, 16, 17, 18, 19, or 20.

In formula (A), when each of R^{a}, R^{b}, and/or R^{c} is a heterocyclic group (that may have one or more substituents), the total number of carbon atoms and hetero atoms in the heterocyclic group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the heterocyclic structure, but may be 3 or more, for example 4 or more, or 5 or more. Specific examples of the number of atoms include 3, 4, 5, 6, 7, 8, 9, 10, 11, 12, 13, 14, 15, 16, 17, 18, 19, or 20.

In formula (A), when each of R^{a}, R^{b}, and/or R^{c} is a metalloxy group (that may have one or more substituents), i.e., a group represented by formula (R)ₙ-M-O-, the type of the group may preferably be, although is not particularly limited to, a group where M is aluminum (Al) (i.e., aluminum oxy group). When each of R^{a}, R^{b}, and/or R^{c} is an aluminum oxy group, n in the formula may be typically 2. A compound represented by formula (A) having such an aluminum oxy group may be generated via a reaction involving a plurality of compounds represented by formula (A) in the amination reaction system. For example, in the case of compounds represented by formula (A) in which M is aluminum (Al) (which may be represented by "R₃Al," where R^{a}, R^{b}, and R^{c} are collectively referred to as "R" without distinction), it is deemed that such compounds cause R₂Al-OH in the system, which compound then reacts with unreacted R₃Al to cause R₂Al-O-AlR₂. This compound R₂Al-O-AlR₂ also corresponds to a compound represented by formula (A), if the substituent aluminum oxy group R₂Al-O- is considered to be a substituent R. Although not bound by any theory, the present inventors speculate that the formation of such a compound R₂Al-O-AlR₂ in the system is one of the reasons for the excellent reaction efficiency in the production method of the present invention.

Among them, each of R^{a}, R^{b}, and/or R^{c} in formula (A) may preferably be, independently of each other, an alkyl group, alkenyl group, alkylyl group, cycloalkyl group, alkoxy group, aryl group, aryloxy group, acyl group, heterocyclic group, heterocyclic oxy group, or metalloxy group that may have one or more substituents.

Specific examples of R^{a}, R^{b}, and/or R^{c} in formula (A) may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethylamino group, benzylamino group, and triphenylmethylamino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group;
*Heterocyclic oxy groups such as furanyl oxy group, pyrrolyl oxy group, indolyl oxy group, and quinolyl oxy group;
*Groups derived from any of the groups mentioned above via substitution with 1 or more substituents (e.g., halogens); and
*Substitution aluminum oxy groups R₂Al-O-, which have any of the groups mentioned above as substituents R.

In the amidation reaction agent of the present invention, the aluminum compounds represented by formula (A) may be used either singly or in combination of any two or more at any ratios.

The amidation reaction agent of the present invention, which employs aluminum compounds represented by formula (A), makes it possible to cause amidation reactions between terminal carboxyl groups of various amino acid or peptide compounds represented by formula (R1) and terminal amino groups of various amino acid ester or peptide ester compounds represented by formula (R2) in a highly efficient and/or highly stereoselective manner, to thereby produce peptide compounds represented by formula (P1) (the production method (1) of the present invention). In addition, the method also makes it possible to cause, following the reactions mentioned above, amidation reactions between terminal amino groups of the peptide ester compounds represented by formula (P1) and terminal carboxyl groups of amino acids or peptide compounds represented by formula (R3) in a highly efficient and/or highly stereoselective manner, to thereby produce peptide compounds represented by formula (P2) (the production method (2) of the present invention).

Conventionally, C-terminal unprotected amino acids and peptide compounds have been used as electrophilic species in amidation reactions, while C-terminal esterified amino acid esters and peptide esters have been used as nucleophiles in general, since they have higher solubility in organic solvents, and are relatively stable, than C-terminal unprotected amino acids. However, their usefulness as electrophilic species has not been focused on because their C-terminals are strongly blocked. In contrast, the amidation reaction agent of the present invention, which employ aluminum compounds represented by formula (A), makes it possible to produce peptide compounds of formula (P2) by causing amidation reactions with nucleophilic amino acid ester or peptide compounds of formula (R2), even when amino acid ester or peptide esters with esterified C-terminal ends are used as the electrophilic compounds of formula (R1).

These reactions using the amidation reaction agent of the present invention may not require metal catalysts and other additives, which have been used in conventional amidation. In addition, depending on the substrate compounds and reaction conditions, these reactions may not even require solvents (reaction medium), making the isolation and purification of products after reaction much easier. However, the present invention does not preclude the use of metal catalysts, additives, or solvents in any way, and there are cases where reaction efficiency and stereoselectivity can be further improved by the use of some additives (e.g., silane compounds described below) or solvents.

### [III. Production Method (1) of Peptide Compounds]

### * Summary

Another embodiment according to the present invention relates to a method for producing a polypeptide compound, the method including causing an amide forming reaction between the carboxyl group on the right side of an amino acid or peptide compound represented by formula (R1) and the amino group on the left side of represented by formula (R2) amino acid ester or peptide ester compound in the presence of an aluminum compound represented by formula (A), to produce a peptide compound represented by formula (P1) (hereinafter also referred to as "the production method (1) of peptide compounds according to the present invention" or simply as "the production method (1) of the present invention").

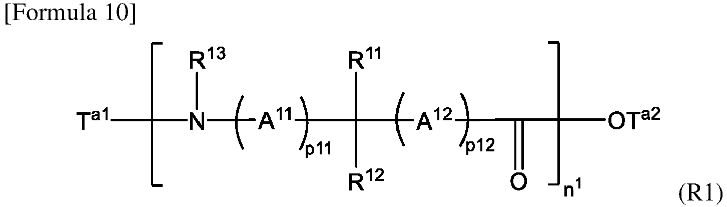

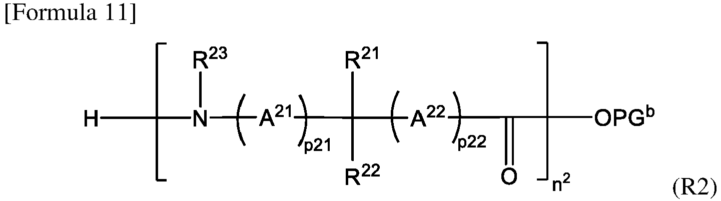

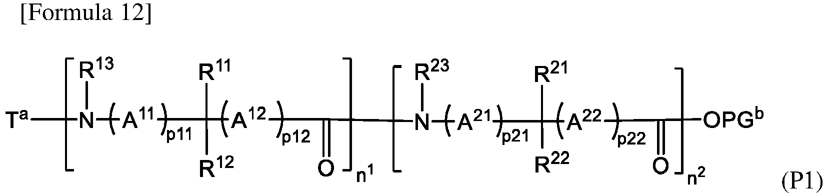

### *Substrate Compounds

The symbols in formulae (R1) and (R2) are defined as follows.

R¹¹, R¹², R²¹, and R²² each represent, independently of each other, a hydrogen atom, halogen atom, hydroxyl group, carboxyl group, nitro group, cyano group, or thiol group, or a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

When each of R¹¹, R¹², R²¹, and/or R²² is a monovalent hydrocarbon group or heterocyclic group that may have one or more substituents, a linking group may intervene between the hydrocarbon group or heterocyclic group and the carbon atom to which it binds. The linking group may be, independently of each other, selected from, although is not limited to, the structures listed below (where, in the chemical formulae below, A represents, independently of each other, a monovalent hydrocarbon group or heterocyclic group that may have one or more substituents. When two A's are present in the same group, they may be identical to each other or different from each other.).

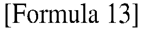

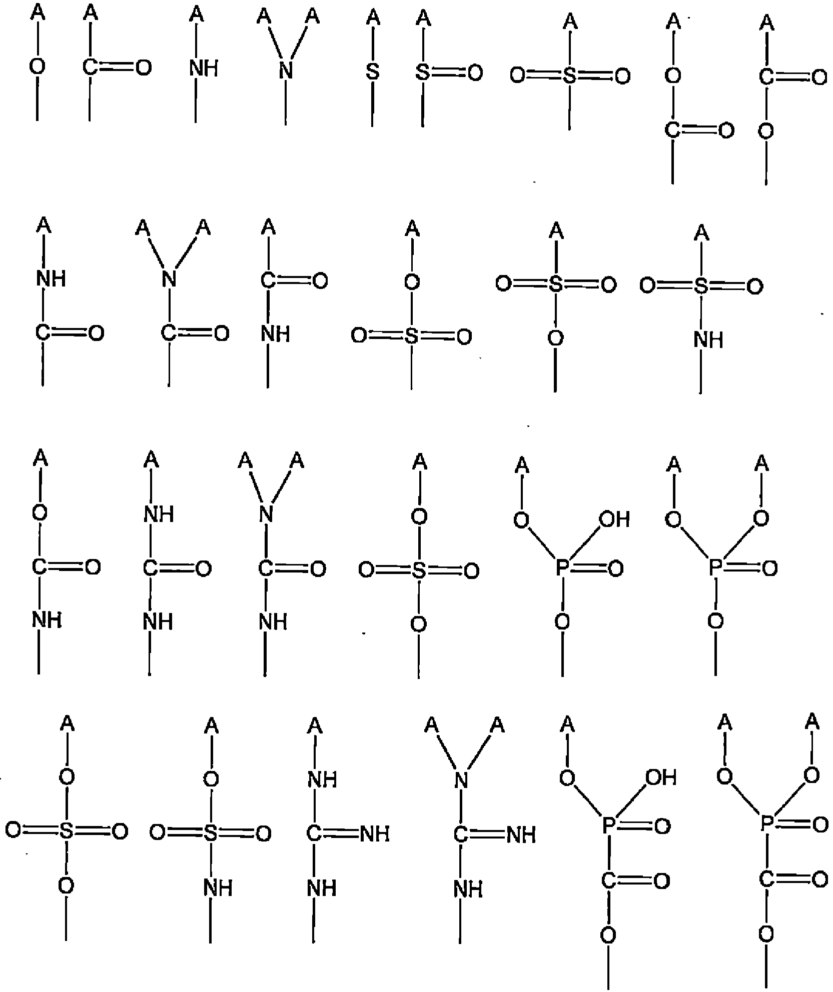

The number of carbon atoms in the hydrocarbon group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The total number of carbon atoms and hetero atoms in the heterocyclic group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the heterocyclic structure, but may be 3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Among them, each of R¹¹, R¹², R²¹, and R²² may preferably be, independently of each other, a hydrogen atom, hydroxyl group, thiol group, carboxyl group, nitro group, cyano group, or halogen atom, or an amino group, alkyl group, alkenyl group, cycloalkyl group, alkoxy group, aryl group, aryloxy group, acyl group, heterocyclic group, or heterocyclic oxy group that may have one or more substituents.

Specific examples of R¹¹, R¹², R²¹, and R²² may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethylamino group, benzylamino group, and triphenylmethylamino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyl oxy group, pyrrolyl oxy group, indolyl oxy group, and quinolyl oxy group.

Of the groups mentioned above, those having a carboxyl group may or may not have a protective group. Although it depends on the reactivity of the compound of formula (R1) and the compound of formula (R2) used in the reaction, if the carboxyl group in any of the above substituents has a protective group, the reaction selectivity with the carboxyl ester group on the right side of the compound represented by formula (R2) may usually be improved over that with the carboxyl group present on the other substituents.

R¹³and R²³ each represent, independently of each other, a hydrogen atom, carboxyl group, or hydroxyl group, or a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

When each of R¹³and/or R²³ is a monovalent hydrocarbon group or heterocyclic group that may have one or more substituents, a linking group may intervene between the hydrocarbon group or heterocyclic group and the nitrogen atom to which it binds. The linking group may be, independently of each other, selected from, although is not limited to, the structures listed below (where, in the chemical formulae below, A represents, independently of each other, a monovalent hydrocarbon group or heterocyclic group that may have one or more substituents. When two A's are present in the same group, they may be identical to each other or different from each other.).

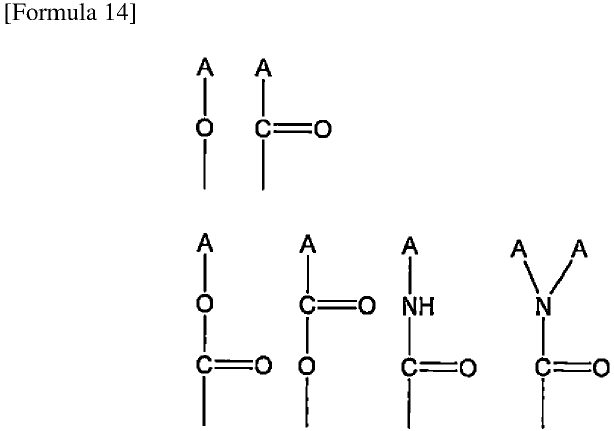

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Each of R¹³and R²³ may preferably be, independently of each other, a hydrogen atom, hydroxyl group, or carboxyl group, or an alkyl group, alkenyl group, cycloalkyl group, alkoxy group, aryl group, aryloxy group, acyl group, heterocyclic group, or heterocyclic oxy group that may have one or more substituents.

Specific examples of R¹³and R²³ may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethyl amino group, benzyl amino group, and triphenylmethyl amino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyl oxy group, pyrrolyl oxy group, indolyl oxy group, and quinolyl oxy group.

Alternatively, R¹¹ and R¹³ may be bound to each other to form, together with the carbon atom to which R¹¹ binds and the nitrogen atom to which R¹³ binds, a hetero ring that may have one or more substituents, and R²¹ and R²³ may be bound to each other to form, together with the carbon atom to which R²¹ binds and the nitrogen atom to which R²³ binds, a hetero ring that may have one or more substituents. If these groups have one or more substituents, they may be selected arbitrarily from those detailed earlier. The number of substituents may be 5, 4, 3, 2, 1, or 0.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

A¹¹, A¹², A²¹, and A²² each represent, independently of each other, a divalent aliphatic hydrocarbon group containing 1 to 3 carbon atoms that may have one or more substituents. Specific Examples include, although are not limited to, methylene group, ethylene group, propylene group, and isopropylene group, as well as groups derived from these groups via substitution with one or more substituents mentioned above. Specific examples of the number of substituents are 3, 2, 1, or 0.

p11, p12, p21, and p22 each represent, independently of each other, 0 or 1.

n¹ represents the number of amino acid units parenthesized with [ ] in formula (R1), and is an integer of 1 or more. When n¹ is 1, the compound of formula (R1) is an amino acid, and when n¹ is 2 or more, the compound of formula (R1) is a peptide. The upper limit for n¹ is not particularly limited so long as the amidation step proceeds, but may preferably be, for example, 100 or less, 80 or less, 60 or less, 50 or less, 40 or less, 30 or less, 20 or less, 15 or less, 12 or less, or 10 or less. Specific examples of n¹ may be 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100.

n² represents the number of amino acid units parenthesized with [ ] in formula (R2), and is an integer of 1 or more. When n² is 1, the compound of formula (R2) is an amino acid, and when n² is 2 or more, the compound of formula (R2) is a peptide. The upper limit for n² is not particularly limited so long as the amidation step proceeds, but may preferably be, for example, 1, 2, 3, 4, 5, 6, 7, 8, 9, or 10. However, n² may preferably be 1, i.e., the compound of formula (R2) may preferably be an amino acid. Although not bound by any theory, the present inventors speculate that when n² is 1, that is, when the compound of formula (R2) is an amino acid, it may react with the aluminum compound of formula (A) to form a cyclization intermediate containing an aluminum atom, which is one factor that improves the reaction efficiency.

Needless to say, when n¹ is equal to or greater than 2, then R¹¹, R¹², R¹³, A¹¹, A¹², p11, and p12, which define the structure units parenthesized with [ ], may be either identical to each other or different from each other between the two or more amino acid units. Likewise, when n² is equal to or greater than 2, then R²¹, R²², R²³, A²¹, A²², p21, and p22, which define the structure units parenthesized with [ ], may be either identical to each other or different from each other between the two or more amino acid units. In other words, when each of the compound of formula (R1) and/or the compound of formula (R2) is a peptide, then the two or more amino acid units constituting the peptide may be either identical to each other or different from each other.

T^{a1} represents a hydrogen atom or monovalent substituent. When it is a monovalent substituent, specific examples may include, although are not limited to, the groups mentioned as examples for R¹³ and R²³ above, as well as protective groups for amino groups (hereinafter also referred to as PG^{a}). The protective group for amino groups PG^{a} is not restricted as long as the amino group can be protected so that it does not react during the amidation reaction and can be deprotected and converted to an amino group after the reaction. The details of the protective groups for amino groups PG^{a} will be explained later.

T^{a2} represents a hydrogen atom or monovalent substituent. When it is a monovalent substituent, specific examples may include, although are not limited to, a monovalent hydrocarbon group or heterocyclic group that may have one or more substituents. When it has one or more substituents, the details of the substituents are as described above, but they may preferably be selected from a halogen atom, hydroxyl group, carboxyl group, nitro group, cyano group, thiol group, sulfonic acid group, amino group, amide group, imino group, or imide group. The number of substituents may be 5, 4, 3, 2, 1, or 0.

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

When T^{a2} is a monovalent substituent, specific examples thereof may include, although are not limited to, the following.

*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Aryl groups such as phenyl group, benzyl group, tolyl group, naphthyl group, and anthracenyl group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group;
*Groups derived from any of the groups mentioned above via substitution of one or more hydrogen atoms with, independently of each other, a substituent such as a halogen atom, hydroxyl group, carboxyl group, nitro group, cyano group, thiol group, sulfonic acid group, amino group, amide group, imino group, or imide group.

PG^{b} represents a protective group for carboxyl groups. protective group for carboxyl groups PG^{b} is not restricted as long as the carboxyl group can be protected so that the carboxyl group concerned does not react in the amidation reaction and can be deprotected and converted to a carboxyl group after the reaction. The details of the protective group for carboxyl groups PG^{b} will be explained later.

In the compound of formula (R2), the amino group on the left side of the formula may form a salt with a counter acid. In this case, examples of the counter acid include, but are not limited to, aliphatic carboxylic acids with 1 to 5 carbons such as acetic acid and propionic acid; trifluoroacetic acid, hydrochloric acid, hydrobromic acid, sulfuric acid, nitric acid, phosphoric acid, boric acid and sulfonic acid.

Any of the above-mentioned substrate compounds (R1) and (R2) may be used either singly or in combination of any two or more compounds at any ratios.

When the group -T^{a} on the C-terminal side of formula (R1) is a monovalent substituent, then the compound of formula (R1) is an amino acid ester or peptide ester with the C-terminus esterified, and also satisfies the definition of the compound of formula (R2). In this case, the amino acid esters or peptide esters of formulae (R1) and (R2), which are substrate compounds, may be either the same compound or different compounds. When multiple different amino acid esters or peptide esters are used as substrate compounds, which one functions as the electrophilic compound (R1) and which functions as the nucleophilic compound (R2) are determined by the following criteria.

1) When there is a difference in reactivity of the ester groups possessed by the multiple amino acid esters or peptide esters, the amino acid ester or peptide ester using the more reactive ester group usually functions as the electrophilic compound (R1), and the amino acid ester or peptide ester using the less reactive ester group functions as the nucleophilic compound (R2). For example, the tert-butyl ester group used in the Examples below is strongly bonded and is not deprotected under the reaction conditions employed in the method of the present invention. Therefore, if one of the multiple amino acid esters or peptide esters has a tert-butyl ester group, that one usually functions as the nucleophilic amino acid.
2) When the aluminum compound of formula (A) is first mixed with one of the multiple amino acid esters or peptide esters and then with the other, the amino acid ester or peptide ester that is mixed with the aluminum compound of formula (A) first functions as the electrophilic compound (R1), and the amino acid ester or peptide ester that is mixed later functions as the nucleophilic compound (R2). This is because the amino acid or peptide ester that is mixed with the aluminum compound of formula (A) first is significantly more susceptible to nucleophilic attack, as its esterified carbonyl group becomes an active ester.

Some or all of either the substrate compound (R1) or (R2) may be linked or immobilized to a carrier such as a basal plate or resin at any of the substituents. In this case, the type of the carrier such as the basal plate or resin is not limited. Any carriers such as basal plates or resins known so far can be used to the extent that they do not substantially interfere with the amide bond reaction in the production method of the present invention and do not depart from the purpose of the present invention. There is no limitation to the means to link or immobilize the substrate compound to the carrier such as the basal plate or resin, but it may be preferable to form a covalent bond between a substituent of the substrate compound and a substituent present on the basal plate, resin, or other carrier. There are also no limitations on the types of each substituent or the method of forming the covalent bond. Any types of substituents and methods for forming a covalent bond known so far can be used, as long as they do not substantially interfere with the amide bond reaction in the production method of the present invention, and to the extent that they do not depart from the purpose of the present invention. The substrate compound may be linked or immobilized to a basal plate, resin, or other carrier via a covalent bond using a carboxyl or amino group possessed by the substrate compound (other than the carboxyl ester or amino group that is the target of the amide bond reaction formation). Such an embodiment can be regarded similarly to an embodiment in which the carboxyl or amino group possessed by the substrate compound (other than the carboxyl ester or amino group that is the target of the amide bond reaction formation) is protected by introducing a protecting group.

### *Protective group for amino groups:

Various protecting groups for amino groups are known to the art. Examples include monovalent hydrocarbon groups that may have one or more substituents and monovalent heterocyclic groups that may have one or more substituents. Preferred among them include monovalent hydrocarbon groups that may have one or more substituents. However, a linking group may intervene between the hydrocarbon group or heterocyclic group and the nitrogen atom of the amino group it protects. The linking group may be, independently of each other, selected from, although is not limited to, the following groups (where, in the chemical formulae below, A represents, independently of each other, a monovalent hydrocarbon group or heterocyclic group that may have one or more substituents. When two A's are present in the same group, they may be identical to each other or different from each other.).

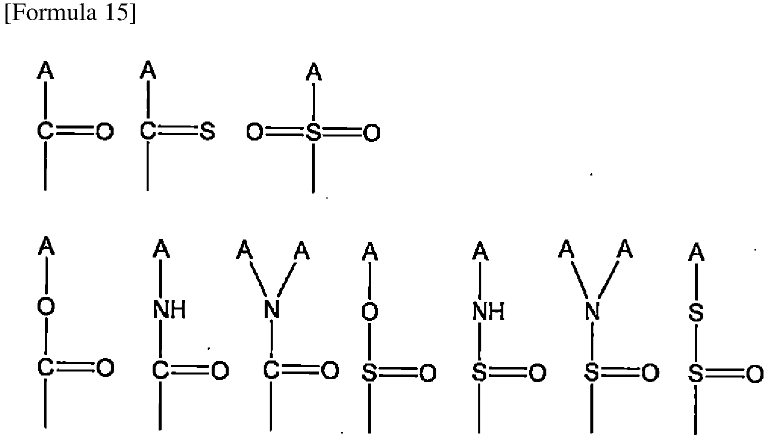

The number of carbons in the protective group may be typically 1 or more, or 3 or more, and typically 20 or less, or 15 or less.

Among them, the amino-protecting group may preferably be one or more selected from the group consisting of monovalent hydrocarbon groups, acyl groups, hydrocarbon oxycarbonyl groups, hydrocarbon sulfonyl groups and amides that may have one or more substituents.

Specific examples of the amino-protective group are listed below. Incidentally, an amino-protective group may be referred to either by the name of the functional group excluding the nitrogen atom of the amino group to which it binds or by the name of the group including the nitrogen atom to which it binds. The following list includes either or both of these names for each protective group.

Examples of unsubstituted or substituted hydrocarbon groups includes: alkyl groups such as methyl group, ethyl group, and propyl group; alkenyl groups such as ethenyl group, propenyl group, and allyl group; alkynyl groups such as propargyl group; cycloalkyl groups such as cyclopropyl group, cyclobutyl group, cyclopentyl group, and cyclohexyl group; aryl groups such as phenyl group, benzyl group, p-methoxybenzyl group, tolyl group, and triphenylmethyl group (Troc group); and substituted hydrocarbon groups such as cyanomethyl group. The number of carbon atoms may typically be 1 or more, or 3 or more, and typically 20 or less, or 15 or less.

Examples of unsubstituted or substituted acyl groups includes: benzoyl group (Bz), o-methoxybenzoyl group, 2,6-dimethoxy benzoyl group, p-methoxybenzoyl group (PMPCO), cinnamoyl group, and phthaloyl group (Phth).

Examples of unsubstituted or substituted hydrocarbon oxycarbonyl groups includes: tert-butoxycarbonyl group (Boc), benzyloxycarbonyl group (Cbz or Z), methoxycarbonyl group, ethoxycarbonyl group, 2-(trimethylsilyl)ethoxycarbonyl group, 2-phenyl ethoxycarbonyl group, 1-(1-adamanthyl)-1-methylethoxycarbonyl group, 1-(3,5-di-t-butylphenyl)-1-methylethoxycarbonyl group, vinyloxycarbonyl group, allyloxycarbonyl group (Alloc), N-hydroxypiperidinyloxycarbonyl group, p-methoxybenzyloxycarbonyl group, p-nitrobenzyloxycarbonyl group, 2-(1,3-dithianyl)methoxycarbonyl, m-nitrophenoxycarbonyl group, 3,5-dimethoxybenzyloxycarbonyl group, o-nitrobenzyloxycarbonyl group, 2,2,2-trichloroethoxycarbonyl group (Troc), and 9-fluorenylmethyloxycarbonyl group (Fmoc).

Examples of unsubstituted or substituted hydrocarbon sulfonyl groups includes: methane sulfonyl group (Ms), toluenesulfonyl group (Ts), and 2- or 4-nitro benzene sulfonyl (Ns) group.

Examples of unsubstituted or substituted amide groups includes: acetamide, o-(benzoyloxymethyl)benzamide, 2-[(t-butyl-diphenyl-siloxy)methyl]benzamide, 2-toluenesulfonamide, 4-toluenesulfonamide, 2-nitro benzene sulfonamide, 4-nitro benzene sulfonamide, tert-butylsulfinyl amide, 4-toluenesulfonamide, 2-(trimethylsilyl)ethane sulfonamide, and benzyl sulfonamide.

In terms of deprotection methods, the protective group may be deprotected by, e.g., at least one of the following methods: deprotection by hydrogenation, deprotection by weak acid, deprotection by fluorine ion, deprotection by one-electron oxidizing agent, deprotection by hydrazine, and deprotection by oxygen.

Preferred examples of the amino protective group include mesyl group (Ms), tert-butoxycarbonyl group (Boc), benzyl group (Bn or Bzl), benzyloxycarbonyl group (Cbz), benzoyl group (Bz), p-methoxybenzyl group (PMB), 2,2,2-trichloroethoxycarbonyl group (Troc), allyloxycarbonyl group (Alloc), 2,4-dinitrophenyl group (2,4-DNP), phthaloyl group (Phth), p-methoxybenzoyl group (PMPCO), cinnamoyl group, toluenesulfonyl group (Ts), 2- or 4-nitrobenzenesulfonyl group (Ns), cyanomethyl group, and 9-fluorenylmethyloxycarbonyl group (Fmoc). These protective groups are preferred because, as mentioned above, they can easily protect the amino group and can be removed under relatively mild conditions.

More preferable examples of the amino protective group include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, cinnamoyl group, 2- or 4-nitrobenzenesulfonyl group (Ns), toluenesulfonyl group (Ts), phthaloyl group (Phth), 2,4-dinitrophenyl group (2,4-DNP), and 9-fluorenylmethyloxycarbonyl group (Fmoc).

### *Protective group for carboxyl groups:

Various protective groups for carboxyl groups are known to the art. Examples include a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Specific examples of protective group for carboxyl groups may include, although are not limited to, the following.

*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Aryl groups such as phenyl, benzyl, tolyl, naphthyl and anthracenyl groups;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
*Silicon-based protective groups such as trimethylsilyl (TMS) group, triethylsilyl (TES) group, triisopropylsilyl (TIPS) group, tri(tert-butyl)silyl (TBS) group, tert-butyldiphenylsilyl (TBDPS) group and tris(trialkylsilyl)silyl group.

### *Silane compounds:

In the production method (1) of the present invention, a silane compound may be coexisted in the reaction system. Carrying out the reaction with a silane compound in the reaction system may result in various advantages such as improved reaction yield and stereo selectivity.

Examples of silane compounds include: various tris{halo-(preferably fluorine-substituted alkyl}silanes such as HSi(OCH(CF₃)₂)₃, HSi(OCH₂CF₃)₃, HSi(OCH₂CF₂CF₂H)₃, HSi(OCH₂CF₂CF₂CF₂CF₂H)₃; as well as trimethylsilyl trifluoromethanesulfonate(TMS-OTf), 1-(trimethylsilyl)imidazole (TMSIM), dimethyl ethylsilyl imidazole (DMESI), dimethyl isopropylsilyl imidazole (DMIPSI), 1-(tert-butyl dimethylsilyl)imidazole (TBSIM), 1-(trimethylsilyl)triazole, 1-(tert-butyl dimethylsilyl)triazole, dimethylsilyl imidazole, dimethylsilyl (2-methyl)imidazole, trimethyl bromosilane (TMBS), trimethyl chlorosilane (TMCS), N-methyl-Ntrimethylsilyl trifluoroacetamide (MSTFA), N,O-bis(trimethylsilyl)trifluoroacetamide (BSTFA), N,O-bis(trimethylsilyl)acetamide (BSA), N-(trimethylsilyl)dimethyl amine (TMSDMA), N-(tert-butyl dimethylsilyl)-N-methyl trifluoroacetamide (MTBSTFA), and hexamethyldisilazane (HMDS). Any one of these may be used alone, or two or more may be used together in any combination and ratio.

### *Lewis acid catalyst:

In the production method (1) of the present invention, the reaction system may also contain a Lewis acid catalyst. Carrying out the reaction with a Lewis acid catalyst in the reaction system may lead to various advantages, such as improved reaction yield and stereoselectivity. On the other hand, however, when a Lewis acid catalyst is used, it may be necessary to separate and remove the Lewis acid catalyst from the reaction product. Therefore, it is preferable to determine whether to use a Lewis acid catalyst taking into consideration the purpose of using the production method according to the present invention.

When a Lewis acid catalyst is used in the production method of the present invention, the type of catalyst is not limited, but it may preferably be a metal compound that functions as a Lewis acid. Examples of metal elements constituting the metal compound include various metals belonging to groups 2 through 15 of the periodic table. Examples of such metal elements include boron, magnesium, gallium, indium, silicon, calcium, lead, bismuth, mercury, transition metals, and lanthanoid elements. Examples of transition metals include scandium, titanium, vanadium, chromium, manganese, iron, cobalt, nickel, copper, zinc, yttrium, zirconium, niobium, molybdenum, technetium, ruthenium, rhodium, palladium, tin, silver, cadmium, hafnium, tantalum, tungsten, rhenium, osmium, iridium, platinum, gold, and thallium. Examples of lanthanoid elements include lanthanum, cerium, neodymium, samarium, europium, gadolinium, holmium, erbium, thulium, ytterbium. From the viewpoint of excellent reaction acceleration and highly stereoselective production of amide compounds, the metal element may preferably be one or more selected from titanium, zirconium, hafnium, tantalum, niobium, boron, vanadium, tungsten, neodymium, iron, lead, cobalt, copper, silver, palladium, tin, and thallium, more preferably one or more selected from titanium, zirconium, hafnium, tantalum, and niobium. The metal compound may contain one, two or more metal atoms. If the metal compound contains two or more metal atoms, the two or more metal atoms may be either of the same metal element or of different metal elements.

Ligands constituting the metal compound may be selected according to the type of the metal element. Examples of ligands include: substituted or unsubstituted linear- or branched-chain alkoxy groups containing 1 to 10 carbon atoms, such as methoxy group, ethoxy group, propoxy group, butoxy group, trifluoroethoxy group, and trichloroethoxy group; halogen atoms such as fluorine atom, chlorine atom, bromine atom, iodine atom; aryloxy groups having 1 to 10 carbon atoms; acetylacetonate group (acac), acetoxy group (AcO), trifluoromethane sulfonate group (TfO); substituted or unsubstituted linear- or branched-chain alkyl groups having 1 to 10 carbon atoms; phenyl group, oxygen atom, sulfur atom, group -SR (where R represents a substituent exemplified by substituted or unsubstituted hydrocarbon groups containing 1 to 20 carbon atoms), group -NRR' (where R and R', independently of each other, represent a hydrogen atom or a substituent exemplified by substituted or unsubstituted hydrocarbon groups containing 1 to 20 carbon atoms), and cyclopentadienyl (Cp) group.

Preferred metal compounds among these are titanium compounds, zirconium compounds, hafnium compounds, tantalum compounds, or niobium compounds. Examples of these metal compounds are indicated below. Any one of these may be used alone, or two or more may be used together in any combination and ratio.

Examples of titanium compounds include those represented by TiX¹₄ (where 4 X¹'s, independently of each other, represent any of the ligands exemplified above, provided that 4 X¹'s may be the same type of ligand or different from each other.). When X¹ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X¹ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X¹ is a halogen atom, it may preferably be a chlorine atom or a bromine atom. Preferred examples of titanium compounds include Ti(OMe)₄, Ti(OEt)₄, Ti(OPr)₄, Ti(Oi-Pr)₄, Ti(OBu)₄, Ti(Ot-Bu)₄, Ti(OCH₂CH(Et)Bu)₄, CpTiCl₃, Cp₂TiCl₂, Cp₂Ti(OTf)₂, (i-PrO)₂TiCl₂, and (i-PrO)₃TiCl.

Examples of zirconium compounds include those represented by ZrX²₄ (where 4 X²'s, independently of each other, represent any of the ligands exemplified above, provided that 4 X²'s may be the same type of ligand or different from each other.). When X² is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X² is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X² is a halogen atom, it may preferably be a chlorine atom or a bromine atom. Preferred examples of zirconium compounds include Zr(OMe)₄, Zr(OEt)₄, Zr(OPr)₄, Zr(Oi-Pr)₄, Zr(OBu)₄, Zr(Ot-Bu)₄, Zr(OCH₂CH(Et)Bu)₄, CpZrCl₃, Cp₂ZrCl₂, Cp₂Zr(OTf)₂, (i-PrO)₂ZrCl₂, and (i-PrO)₃ZrCl.

Examples of hafnium compounds include those represented by HfX³₄ (where 4 X³'s, independently of each other, represent any of the ligands exemplified above, provided that 4 X³'s may be the same type of ligand or different from each other.). When X³ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X³ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X³ is a halogen atom, it may preferably be a chlorine atom or a bromine atom. Preferred examples of hafnium compounds include HfCp₂Cl₂, HfCpCl₃, and HfCl₄.

Examples of tantalum compounds include those represented by TaX⁴₅ (where 5 X⁴'s, independently of each other, represent any of the ligands exemplified above, provided that 5 X⁴'s may be the same type of ligand or different from each other.). When X⁴ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁴ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁴ is a halogen atom, it may preferably be a chlorine atom or a bromine atom. Preferred examples of tantalum compounds include tantalum alkoxide compounds (e.g., compounds in which X⁴ is an alkoxy group) such as Ta(OMe)₅, Ta(OEt)₅, Ta(OBu)₅, Ta(NMe₂)₅, Ta(acac)(OEt)₄, TaCl₅, TaCl₄(THF), and TaBrs. Other examples are those in which X⁴ is an oxygen, such as Ta₂O₅.

Examples of niobium compounds include those represented by NbX⁵₅ (where 5 X⁵'s, independently of each other, represent any of the ligands exemplified above, provided that 5 X⁵'s may be the same type of ligand or different from each other.). When X⁵ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁵ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁵ is a halogen atom, it may preferably be a chlorine atom or a bromine atom. Preferred examples of niobium compounds include niobium alkoxide compounds (e.g., compounds in which X⁵ is an alkoxy group) such as NbCl₄(THF), NbCl₅, Nb(OMe)₅, and Nb(OEt)₅. Other examples are those in which X⁵ is an oxygen, such as Nb₂O₅.

The Lewis acid catalyst may be loaded on a carrier. There are no particular restrictions on the carrier on which the Lewis acid catalyst is to be loaded, and any known carrier can be used. Also, any known method can be used to load the Lewis acid catalyst on the carrier.

### *Other ingredients:

In the production method (1) of the present invention, any other ingredients may coexist in the system in addition to the substrate compounds, i.e., the amino acid or peptide compound of formula (R1) and the amino acid ester or peptide ester compound of formula (R2); the amidation reaction agent, i.e., the aluminum compounds represented by formula (A); and a silane compound and/or a Lewis acid catalyst, which may optionally be used. Examples of other ingredients may include, although are not limited to, conventional catalysts (other than Lewis acid catalysts) that can be used for an amidation reaction, bases, phosphorus compounds, and solvents. Any one of these may be used alone, or two or more may be used together in any combination and ratio.

Examples of catalysts (other than Lewis acid catalysts) include methylaluminum bis(4-bromo-2,6-di-tert-butylphenoxyde (MABR), trimethylsilyl trifluoromethanesulfonate (TMS-OTf), and methylaluminum bis(2,6-di-tert-butylphenoxyde (MAD). Any one of these may be used alone, or two or more may be used together in any combination and ratio.

The type of base is not restricted, and any base that is known to improve reaction efficiency can be used. Examples of such bases include amines having 1 to 4 linear or branched-chain alkyl groups with 1 to 10 carbons, such as tetrabutylammonium fluoride (TBAF), triethylamine (Et₃N), diisopropylamine (i-Pr₂NH), and diisopropylethylamine (i-Pr₂EtN), as well as inorganic bases such as cesium fluoride. Any one of these may be used alone, or two or more may be used together in any combination and ratio.

Examples of phosphorus compounds include: phosphine compounds such as trimethyl phosphine, triethyl phosphine, tripropyl phosphine, trimethyloxyphosphine, triethyloxyphosphine, tripropyloxyphosphine, triphenyl phosphine, trinaphthyl phosphine, triphenyloxyphosphine, tris(4-methylphenyl)phosphine, tris(4-methoxy phenyl)phosphine, tris(4-fluorophenyl)phosphine, tris(4-methylphenyloxy)phosphine, tris(4-methoxy phenyloxy)phosphine, and tris(4-fluorophenyloxy)phosphine; phosphate compounds such as trimethyl phosphate, triethyl phosphate, tripropyl phosphate, trimethyloxyphosphate, triethyloxyphosphate, tripropyloxyphosphate, triphenyl phosphate, trinaphthyl phosphate, triphenyloxyphosphate, tris(4-methylphenyl)phosphate, tris(4-methoxy phenyl)phosphate, tris(4-fluorophenyl)phosphate, tris(4-methylphenyloxy)phosphate, tris(4-methoxy phenyloxy)phosphate, and tris(4-fluorophenyloxy)phosphate; multivalent phosphine compounds or multivalent phosphate compounds such as 2,2'-bis(diphenylphosphino)-1,1'-binaphthyl (BINAP) and 5,5'-bis(diphenylphosphino)-4,4'-bi-1,3-benzodioxole (SEGPHOS). Any one of these may be used alone, or two or more may be used together in any combination and ratio.

From the viewpoint of increasing reaction efficiency, the reaction may be carried out in a solvent. Examples of solvents include, although are not limited to, aqueous solvents and organic solvents. Examples of organic solvents may include, although are not limited to: aromatic hydrocarbons such as toluene and xylene; eters such as pentane, petroleum ether, tetrahydrofuran (THF), 1-methyl tetrahydrofuran (1-MeTHF), diisopropyl ether (i-Pr₂O), diethyl ether (Et₂O), and cyclopentylmethyl ether (CPME); nitrogen-containing organic solvents acetonitrile (MeCN); chlorine-containing organic solvents such as dichloromethane (DCM); esters such as ethyl acetate (AcOEt); and organic acids such as acetic acids. Any one of these solvents may be used alone, or two or more may be used together in any combination and ratio.

### *Reaction procedure:

In the production method (1) of the present invention, the substrate compounds, i.e., the amino acid or peptide compound of formula (R1) and the amino acid ester or peptide ester compound of formula (R2), may be mixed with the amidation reaction agent, i.e., the aluminum compounds represented by formula (A), to cause a reaction. When a silane compound, a Lewis acid catalyst, and/or other ingredients (e.g., a catalyst other than Lewis acid catalysts, a base, and/or a phosphorus compound) are used as optional ingredients, they may be mixed with the substrate compounds and the amidation reaction agent. When a solvent is optionally used, the above ingredients may be added to the solvent and mixed in the solvent.

For each of the above ingredients, the entire amount may be added to the system all at once, separately in several portions, or continuously in small amounts.

### *Ratios of the amounts of the ingredients used:

In the production method (1) of the present invention, the amount of each component used is not limited, but may preferably be as follows.

The amount ratio of the amino acid or peptide compound of formula (R1) to the amino acid ester or peptide ester compound of formula (R2) is not restricted, but relative to 1 mol of the compound of formula (R1), the compound of formula (R2) may be used in an amount within a range of 0.1 mol or more, or 0.2 mol or more, or 0.3 mol or more, or 0.4 mol or more, or 0.5 mol or more, and 20 mol or less, or 10 mol or less, or 5 mol or less, or 4 mol or less, or 3 mol or less. It may be preferably to use the compound of formula (R2) in a larger amount than the amount of the compound of formula (R1), from the viewpoint of increasing the reaction efficiency. Specifically, relative to 1 mol of the compound of formula (R1), the compound of formula (R2) may be used in an amount of about 2 mol. Needless to say, it is necessary to use at least 1 mol each of the compound of formula (R1) and the compound of formula (R2) as substrates for the target production volume of the compound of formula (P1) to be manufactured.

The amount of the aluminum compounds represented by formula (A) to be used is not restricted as long as it can induce the formation reaction of a peptide compound of formula (P1) from an amino acid or peptide compound of formula (R1) and an amino acid ester or peptide ester compound of formula (R2) through the practice of the production method of the present invention. For example, relative to 1 mol of the compound of formula (R1), the aluminum compound represented by formula (A) may be used in an amount within a range of 0.1 mol or more, or 0.2 mol or more, or 0.3 mol or more, or 0.4 mol or more, or 0.5 mol or more, and 20 mol or less, or 10 mol or less, or 5 mol or less, or 4 mol or less, or 3 mol or less. When two or more aluminum compounds represented by formula (A) are used in combination, the total amount of the two or more aluminum compounds of formula (A) may satisfy the amount ranges mentioned above.

When a silane compound is used, the amount used is not restricted, but when the amount of the compound of formula (R1) is 100 mol %, the silane compound may be used in an amount of 0.1 mol % or more, or 0.2 mol % or more, or 0.3 mol % or more, or 50 mol % or less, or 30 mol % or less, or 2 0 mol % or less, or 15 mol % or less.

When a Lewis acid catalyst is used, the amount used is not restricted, but when the amount of the compound of formula (R1) is 100 mol%, the Lewis acid catalyst may be used in an amount of 0.1 mol% or more, or 0.2 mol% or more, or 0.3 mol% or more, or 50 mol% or less, or 30 mol% or less, or 20 mol% or less, or 15 mol% or less.

When other optional ingredients are used, their amounts should be adjusted accordingly, referring, for example, to the previous findings of the inventor and others in previous patents (Patent Literatures 1 to 6).

### *Reaction conditions:

In the production method (1) of the present invention, the reaction conditions are not restricted as long as the reaction proceeds, but may be exemplified as follows.

The reaction temperature is not restricted as long as the reaction proceeds, but may be 0 °C or more, or 10 °C or more, or 20 °C or more, and 100 °C or less, or 80 °C or less, or 60 °C or less.

The reaction pressure is not restricted as long as the reaction proceeds, and the reaction may be carried out under reduced, normal, or pressurized pressure, but may typically be carried out under normal pressure.

The reaction atmosphere is also not limited as long as the reaction proceeds, but the reaction may be carried out under an atmosphere of inert gas such as argon or nitrogen.

The reaction time is also not limited as long as the reaction proceeds. However, in order for the reaction to proceed sufficiently and efficiently, the reaction time may be 10 minutes or more, or 20 minutes or more, or 30 minutes or more, and for 80 hours or less, or for 60 hours or less, or for 50 hours or less.

The production method (1) of the present invention may be carried out in a sequential manner (batch) or in a continuous manner (flow). Details of specific procedures for implementing a sequential method (batch method) and a continuous method (flow method) are well known in the art.

### *Post-treatments (e.g., purification and recovery):

The peptide compound (P1) produced by the production method (1) of the present invention may be subjected to various post-treatments. For example, the peptide compound (P1) produced can be isolated and purified according to conventional methods such as column chromatography and recrystallization. In addition, when the peptide compound (P1) produced has an amino group and/or a carboxyl group each protected by a protective group, deprotection can be performed according to the method described below. The generated peptide compound (P1) may be subjected, either directly or after isolation and purification, to the later stage process of the production method (2) of the present invention described below to produce a polypeptide with further elongated amino acid residues.

### [IV. Production Method (2) of Peptide Compounds]

### * Summary

Another embodiment of the present invention relates to a method for producing a polypeptide compound, wherein the method includes, as step (i), carrying out the production method (1) of the present invention, and as step (ii), causing an amide forming reaction between the carboxyl group on the right side of an amino acid or peptide compound represented by formula (R3) and the amino group on the left side of the peptide ester compound represented by formula (P1), to thereby produce a peptide compound represented by formula (P2) (hereinafter also referred to as "the production method (2) of peptide compounds according to the present invention" or simply as "the production method (2) of the present invention").

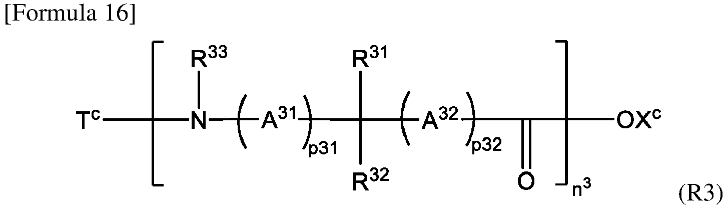

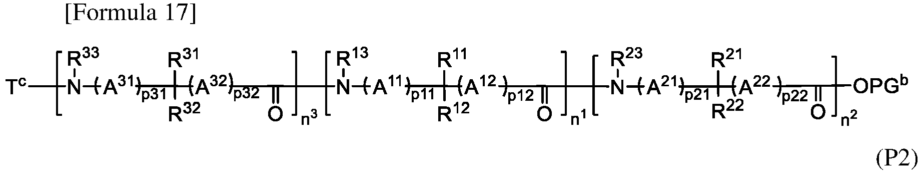

The production method (2) of the present invention makes it possible to modify the peptide ester compound of formula (P1) produced by the production method (1) of the present invention by linking the N-terminal amino group of the peptide ester compound of formula (P1) to the terminal carboxyl group of the amino acid or peptide compound of formula (R3) via an amide forming reaction, to thereby produce the polypeptide compound of formula (P2) with elongated amino acid residues.

In the production method (2) of the present invention, the first step (i) is identical to the production method (1) of the present invention described above. Therefore, the latter step (ii) is mainly explained in the following description.

### *Substrate compounds

The symbols in formula (R3) are defined as follows.

In formula (R3), X^{c} represents a halogen atom, hydroxyl group, or a monovalent aliphatic hydrocarbon group or aromatic hydrocarbon group that may have one or more substituents. According to an embodiment, X^{c} is a halogen atom such as a fluorine, chlorine, bromine, or iodine atom. According to an embodiment, X^{c} is a chlorine atom.

In formula (R3), T^{c} represents a hydrogen atom or monovalent substituent. The details of T^{c} are the same as those explained for T^{a1} of formula (R1) above.

In formula (R3), R³¹and R³² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents. R³³ represents a hydrogen atom, carboxyl group, hydroxyl group, or a monovalent aliphatic hydrocarbon group, aromatic hydrocarbon group, or heterocyclic group that may have one or more substituents. When R³³ is a monovalent aliphatic hydrocarbon group, aromatic hydrocarbon group, or heterocyclic group, it may bind to the nitrogen atom via a linking group. Alternatively, R³¹ and R³³ may be bound to each other to form, together with the carbon atom to which R³¹ binds and the nitrogen atom to which R³³ binds, a heterocyclic group that may have one or more substituents. The details of R³¹, R³², and R³³ are the same as those explained for R¹¹, R¹², and R¹³ of formula (R1) above.

In formula (R3), A³¹and A³² each represent, independently of each other, a divalent aliphatic hydrocarbon group that may have one or more substituents having 1 to 3 carbon atoms. The details of A³¹and A³² are the same as those explained for A¹¹and A¹² of formula (R1) above.

In formula (R3), p31 and p32 each represent, independently of each other, 0 or 1.

In formula (R3), n³ represents an integer of equal to or greater than 1 corresponding to the number of the structure units parenthesized with [ ]. When n³ is 1, the compound of formula (R3) is an amino acid, while when n³ is 2 or more, the compound of formula (R3) is a peptide. The definition of n³ is the same as that explained for n¹ of formula (R1) above.

The substrate compounds (R3) explained above may be used either singly or in combination of any two or more compounds at any ratios.

Some or all of the substrate compound (R3) explained above may be linked or immobilized to a basal plate, resin, or other carrier via any substituent. The details thereof are the same as those explained for the substrate compounds of formulae (R1) and (R2) in the production method (1) of the present invention.

### *Silane compound:

In the production method (2) of the present invention, a silane compound may co-exist in the reaction system silane at step (i) and/or step (ii). The details thereof are the same as those explained for the production method (1) of the present invention.

### *Lewis acid catalyst:

In the production method (2) of the present invention, a Lewis acid catalyst may co-exist in the reaction system silane at step (i) and/or step (ii). The details thereof are the same as those explained for the production method (1) of the present invention.

### *Other ingredients:

In the production method (2) of the present invention, other ingredients (e.g., catalysts other than Lewis acid catalysts, bases, phosphorus compounds, and solvents) may co-exist in the reaction system silane at step (i) and/or step (ii). The details thereof are the same as those explained for the production method (1) of the present invention.

### *Reaction procedure and reaction conditions:

The production method (2) of the present invention may be carried out by, as step (i), mixing the substrate compounds, i.e., the amino acid or peptide compound of formula (R1) and the amino acid ester or peptide ester compound of formula (R2), with the amidation reaction agent, i.e., the aluminum compounds represented by formula (A), to cause a reaction, and then, as step (ii), adding the amino acid or peptide compound of formula (R3) to cause a further reaction. The aluminum compounds represented by formula (A) may be mixed only at step (i), or may also be added as appropriate at step (ii).

After the completion of step (i), it is possible to separate and purify the peptide compound of formula (P1) once generated from the reaction system before mixing it with the amino acid or peptide compound of formula (R3). However, if the peptide compound of formula (P1) is a dipeptide compound (i.e., the substrate compounds (R1) and (R2) are both amino acids), then the compound (P1) may be adsorbed by aluminum hydroxide, etc., generated during the reaction in step (i), making the separation and purification difficult. Therefore, it may be preferable to start step (ii) immediately after the completion of step (i), by adding the amino acid or peptide compound of formula (R3) without separating and purifying the peptide compound of formula (P1). Synthesizing the larger peptide compound of formula (P2) in this manner serves to prevent adsorption by aluminum hydroxide, etc., whereby efficient separation and purification can be achieved.

When optional components such as a silane compound, Lewis acid catalyst, and/or other ingredients (e.g., catalysts other than Lewis acid catalysts, bases, phosphorus compounds, and solvents) are used, they may be added to the reaction system at step (i) and/or step (ii). When a solvent is optionally used, the above ingredients may be added to the solvent and mixed in the solvent.

For each of the above ingredients, the entire amount may be added to the system all at once, separately in several portions, or continuously in small amounts.

### *Ratios of the amounts of the ingredients used:

In the production method (2) of the present invention, the amount of each component used is not limited, but may preferably be as follows.

The amounts of the amino acid or peptide compound of formula (R1) and the amino acid ester or peptide ester compound of formula (R2) and the aluminum compound represented by formula (A) to be used are the same as those explained for the production method (1) of the present invention above. When a silane compound, a Lewis acid catalyst, and/or other optional ingredients are used, the amounts thereof to be used are the same as those explained for the production method (1) of the present invention above.

The amount ratio between the compounds of formulae (R1) and (R2) and the compound of formula (R3) is not restricted, but relative to 1 mol of the compound of formula (R1), the compound of formula (R3) may preferably be used in an amount within the range of 0.1 mol or more, or 0.2 mol or more, or 0.3 mol or more, or 0.4 mol or more, or 0.5 mol or more, and 20 mol or less, or 10 mol or less, or 5 mol or less, or 4 mol or less, or 3 mol or less. It may be preferable to use the compound of formula (R3) in a larger amount than that of the compound of formula (R1), from the viewpoint of increasing the reaction efficiency. Specifically, relative to 1 mol of the compound of formula (R1), the compound of formula (R3) may preferably be used in an amount of about 2 mol. Needless to say, it is necessary to use at least 1 mol each of the compound of formula (R1), the compound of formula (R2), and the compound of formula (R3) as substrates for the target production volume of the compound of formula (P2) to be manufactured.

### *Reaction procedure and reaction conditions:

In the production method (2) of the present invention, the reaction conditions are not restricted as long as the reaction proceeds. Examples of the reaction conditions for the earlier step (i) are the same as those explained for the production method (1) of the present invention above. Examples of the reaction conditions for the later step (ii) are as follows.

The reaction temperature at step (ii) is not restricted as long as the reaction proceeds, but may be 0 °C or more, or 10 °C or more, or 20 °C or more, and 100 °C or less, or 80 °C or less, or 60 °C or less.

The reaction pressure at step (ii) is not restricted as long as the reaction proceeds, and the reaction may be carried out under reduced, normal, or pressurized pressure, but may typically be carried out under normal pressure.

The reaction atmosphere at step (ii) is also not limited as long as the reaction proceeds, but the reaction may be carried out under an atmosphere of inert gas such as argon or nitrogen.

The reaction time of step (ii) is also not limited as long as the reaction proceeds. However, in order for the reaction to proceed sufficiently and efficiently, the reaction time may be 10 minutes or more, or 20 minutes or more, or 30 minutes or more, and for 80 hours or less, or for 60 hours or less, or for 50 hours or less.

Each of steps (i) and (ii) of the production method (2) of the present invention may be carried out in a sequential manner (batch) or in a continuous manner (flow). Details of specific procedures for implementing a sequential method (batch method) and a continuous method (flow method) are well known in the art. Steps (i) and (ii) may be carried out in a continuous manner as a one-pod reaction.

### *Post-treatments (e.g., purification and recovery):

The peptide compound (P2) produced by the production method (2) of the present invention may be subjected to various post-treatments. For example, the peptide compound (P1) produced can be isolated and purified according to conventional methods such as column chromatography and recrystallization. In addition, when the peptide compound (P1) produced has an amino group and/or a carboxyl group each protected by a protective group, deprotection can be performed according to the method described below.

### [V. Others]

The polypeptide compound of formula (P1) or formula (P2) obtained by the production method mentioned above may be subjected to various post-treatments.

For example, the polypeptide compound of formula (P1) or formula (P2) obtained by the production method mentioned above can be isolated and purified according to conventional methods such as column chromatography and recrystallization.

In addition, the polypeptide compound of formula (P1) or formula (P2) obtained by the production method mentioned above can be subjected to deprotection of the amino group protected by the protecting group. The method of deprotecting the protected amino group is not particularly restricted, and various methods can be used depending on the type of the protecting group. Examples include deprotection by hydrogenation, deprotection by weak acids, deprotection by fluorine ions, deprotection by one-electron oxidants, deprotection by hydrazine, and deprotection by oxygen. The deprotection by hydrogenation may be carried out by, e.g.; (a) a method of causing deprotection in the presence of hydrogen gas using a metal catalyst such as palladium, palladium-carbon, palladium hydroxide, palladium-carbon hydroxide, etc., as a reduction catalyst; and (b) a method of causing deprotection in the presence of a metal catalyst such as palladium, palladium-carbon, palladium hydroxide, palladium-carbon hydroxide, etc., using a hydrotreating reductant such as sodium borohydride, lithium aluminum hydride, lithium borohydride, diborane, etc.

Likewise, the polypeptide compound of formula (P1) or formula (P2) obtained by the production method mentioned above can be subjected to deprotection of the carboxyl group protected by the protecting group. The method of deprotecting the protected carboxyl group is not particularly restricted, and various methods can be used depending on the type of the protecting group. Examples include deprotection by hydrogenation, deprotection by bases, and deprotection by weak acids. In the case of deprotection with a base, a strong base such as lithium hydroxide, sodium hydroxide, potassium hydroxide, etc. can be used.

In addition, the polypeptide compound of formula (P1) or formula (P2) obtained by the production method mentioned above may be used (after deprotection and/or replacement of substituents if necessary) as a peptide compound of formula (R1) again for the production method (1) or (2) of the present invention. Alternatively, the polypeptide compound of formula (P1) or formula (P2) obtained by the production method mentioned above may be subjected (after deprotection and/or replacement of substituents if necessary) to other conventionally known amidation or peptide production methods. Thus, a polypeptide compound of formula (P1) or formula (P2) can be linked to other amino acids or peptides by amide bonding to elongate the amino acid residues and synthesize larger polypeptides. Polypeptides of any number of amino acid residues and amino acid sequences can in principle be synthesized by sequentially repeating these steps.

The present inventors have filed the following prior patent applications relating to amidation reactions for linking amino acids or peptides and methods for producing polypeptides thereby. It is possible to perform the various polypeptide production methods of the present invention in combination with the amidation reactions and polypeptide production methods described in these earlier patent applications as appropriate and/or to modify the amidation reactions and polypeptide production methods described in these prior patent applications as appropriate, taking into account the conditions of these prior patent applications. The descriptions of these earlier patent applications are incorporated herein by reference in their entirety.
(1) WO2017/204144 A (May 22, 2017)
(2) WO2018/199146 A (April 25, 2018)
(3) WO2018/199147 A (April 25, 2018)
(4) WO2019/208731 A (April 25, 2019)
(5) WO2021/085635 A (October 30, 2020)
(6) WO2021/085636 A (October 30, 2020)
(7) WO2021/149814 A (January 22, 2021)

### EXAMPLES

The present invention will be described in more detail below with reference to examples. However, the present invention should in no way be bound by the following examples, and can be implemented in any form within the scope that does not depart from the purpose of the invention.

### [Example Group I: Amidation Reactions using Unprotected Amino Acid as Electrophilic Species]

### [Example I-1: Synthesis of dipeptide H-Phe-Ala-OtBu via amidation between H-Phe-OH and H-Ala-OtBt)

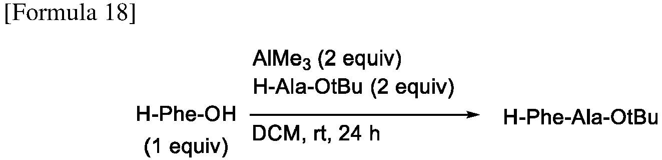

A test tube with a capacity of 20mL containing a stirring bar was charged with phenylalanine (0.25mmol;41.3mg), hexane solution of trimethyl aluminum (AlMe₃) (2mol/L in hexane) (250µL, 2.0 equivalents), and H-Ala-OtBu (70µL, 2 equivalents) in dichloromethane. The mixture was agitated at room temperature for 24 hours. After the reaction, the product was diluted with chloroform (4.50mL), and isolated by silica gel column chromatography, whereby the target compound was obtained (yield: 91%, dr).

### [Example I-2: Synthesis of tripeptide Fmoc-Ala-Phe-Ala-OtBu via amidation between H-Phe-OH, H-Ala-OtBt, and Fmoc-Ala-Cl)

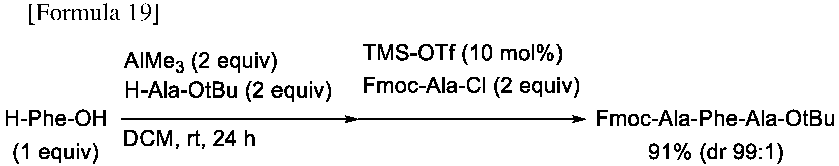

A test tube with a capacity of 20mL containing a stirring bar was charged with phenylalanine (0.25mmol;41.3mg), hexane solution of trimethyl aluminum (AlMe₃) (2mol/L in hexane) (250µL, 2.0 equivalents), and H-Ala-OtBu (70µL, 2 equivalents) in dichloromethane, and the mixture was agitated at room temperature for 24 hours. TMS-OTf (4.5µL, 10 mol %) and Fmoc-Ala-Cl (165mg, 2 equivalents) were added into the test tube, and the mixture was agitated at room temperature further for 24 hours. After the reaction, the product was diluted with chloroform (4.50mL), and isolated by silica gel column chromatography, whereby the target compound was obtained.

### [Example Group II: Amidation Reaction using N-terminal Protected Amino Acid as Electrophilic Species]

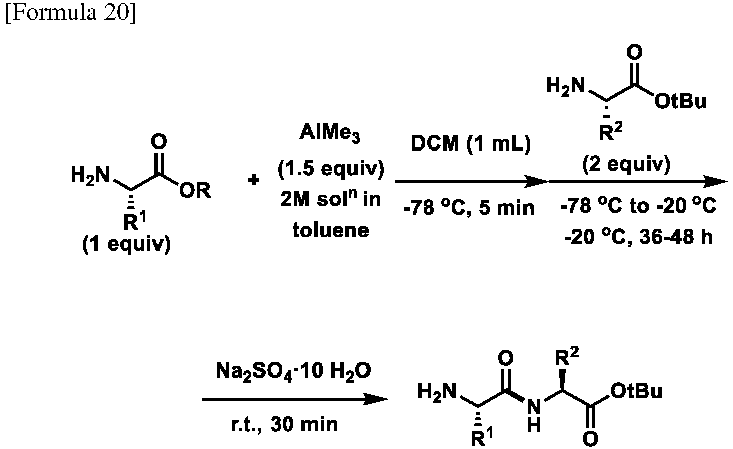

A test tube with a capacity of 20mL containing a stirring bar was charged with amino acid ester (0.25mmol) and hexane solution of trimethyl aluminum (2mol/L in toluene) (187µL, 1.5 equivalents) in dichloromethane, and the mixture was agitated at -78 °C for 5 minutes. After the temperature was elevated to -20 °C, an amino acid tertbutyl ester (2 equivalents) was added into the test tube, and the mixture was agitated at room temperature for 36 to 48 hours. After the reaction, sodium sulfate was added, and the mixture was agitated at room temperature for 30 minutes. After the reaction, the product was diluted with chloroform (4.50mL), and isolated by silica gel column chromatography, whereby the target compound was obtained. The groups -O-R, -R¹, and -R² in the above reaction formula, as well as the yields and dr ratios of the corresponding target compounds are indicated in the table below.

**[Table 3]**

| -O-R | -R¹ | -R² | Yield | dr |
|---|---|---|---|---|
| -O-Me | -CHₛ-Ph | -CH₃ | 89% | >20:1 |
| | -CH₃ | -CH₃ | 79% | >20:1 |
| | 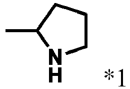 | -CH₃ | 84% | >20:1 |
| -O-Et | -CHₛ-Ph | -CH₃ | 81% | >20:1 |
| -O-iPr | -CHₛ-Ph | -CH₃ | 65% | >20:1 |
| 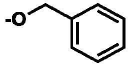 | -CHₛ-Ph | -CH₃ | 91% | >20:1 |
| | -CH₃ | -CH₃ | 77% | >20:1 |
| 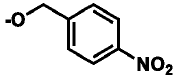 | -CHₛ-Ph | -CH(CH₃)₂ | 60% | >20:1 |
| 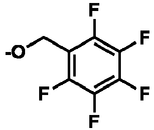 | -CHₛ-Ph | -CH(CH₃)₂ | 60% | >20:1 |
| | -CH₃ | -CH₂CH(CH₃)₂ | 58% | >20:1 |
| 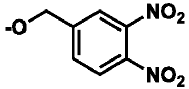 | -CHₛ-Ph | -CH(CH₃)₂ | 94% | >20:1 |
| | -CH₃ | -CH(CH₃)₂ | 79% | >20:1 |
| | -CHₛ-Ph | -CH(CH₃)₂ | 84% | >20:1 |
| | -(CH₂)₄-NHBoc | -CH(CH₃)₂ | 86% | >20:1 |

| | | | | |
|---|---|---|---|---|
| *1: -R¹ is bound to the adjacent amino group to form a pyrrolidine ring. | | | | |

## Claims

1. A method for producing a polypeptide compound, comprising the step of (i) causing an amide forming reaction between the carboxyl group on the right side of an amino acid or peptide compound represented by formula (R1) and the amino group on the left side of represented by formula (R2) amino acid ester or peptide ester compound in the presence of an aluminum compound represented by formula (A), to produce a peptide compound represented by formula (P1). 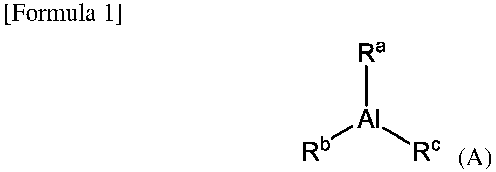
In formula (A),
R^{a}, R^{b}, and R^{c} each represent, independently of each other, a hydrogen atom, halogen atom, hydroxyl group, carboxyl group, nitro group, cyano group, or thiol group, or a monovalent aliphatic hydrocarbon group, aromatic hydrocarbon group, heterocyclic group, aliphatic hydrocarbon oxy group, aromatic hydrocarbon oxy group, heterocyclic group-substituted oxy group, or metalloxy group that may have one or more substituents. 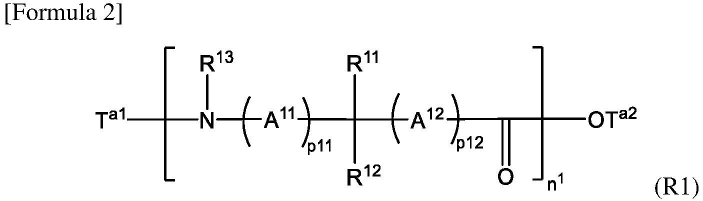
In formula (R1),
T^{a1} and T^{a2} each represent, independently of each other, a hydrogen atom or monovalent substituent,
R¹¹ and R¹² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
R¹³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R¹¹ and R¹³ may be bound to each other to form, together with the carbon atom to which R¹¹ binds and the nitrogen atom to which R¹³ binds, a heterocyclic group that may have one or more substituents,
A¹¹ and A¹² each represent, independently of each other, a divalent aliphatic hydrocarbon group that may have one or more substituents having 1 to 3 carbon atoms,
p11 and p12 each represent, independently of each other, 0 or 1, and
n¹ represents an integer of equal to or greater than 1 corresponding to the number of the structure units parenthesized with [ ], provided that when n¹ is equal to or greater than 2, then the two or more structure units in [ ] may be either identical to each other or different from each other. 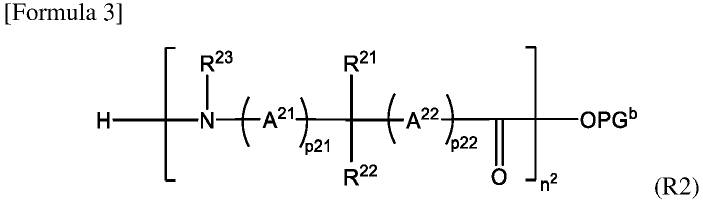
In formula (R2),
PG^{b} represents a protective group for carboxyl groups,
R²¹and R²² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
R²³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R²¹ and R²³ may be bound to each other to form, together with the carbon atom to which R²¹ binds and the nitrogen atom to which R²³ binds, a heterocyclic group that may have one or more substituents,
A²¹ and A²² each represent, independently of each other, a divalent aliphatic hydrocarbon group that may have one or more substituents having 1 to 3 carbon atoms,
p21 and p22 each represent, independently of each other, 0 or 1, and
n² represents an integer of equal to or greater than 1 corresponding to the number of the structure units parenthesized with [ ], provided that when n² is equal to or greater than 2, then the two or more structure units in [ ] may be either identical to each other or different from each other. 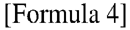 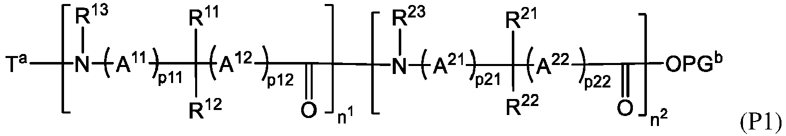
In formula (P1),
T^{a}, R¹¹, R¹², R¹³, A¹¹, A¹², p11, p12, and n¹ each represent the same definitions as those of the same symbols in general formula (R1) above, and
PG^{b}, R²¹, R²², R²³, A²¹, A²², p21, p22, and n² each represent the same definitions as those of the same symbols in general formula (R2) above.

2. The method according to claim 1, further comprising, after step (i), the step of (ii) causing an amide forming reaction between the carboxyl group on the right side of an amino acid or peptide compound represented by formula (R3) and the amino group on the left side of the peptide ester compound represented by formula (P1) to thereby produce a peptide compound represented by formula (P2). 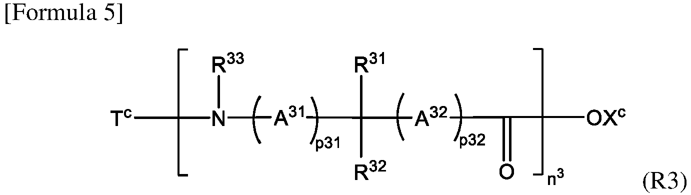
In formula (R3),
T^{c} represents a hydrogen atom or monovalent substituent,
X^{c} represents a halogen atom, hydroxyl group, or a monovalent aliphatic hydrocarbon group or aromatic hydrocarbon group that may have one or more substituents,
R³¹and R³² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
R³³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R³¹ and R³³ may be bound to each other to form, together with the carbon atom to which R³¹ binds and the nitrogen atom to which R³³ binds, a heterocyclic group that may have one or more substituents,
A³¹ and A³² each represent, independently of each other, a divalent aliphatic hydrocarbon group that may have one or more substituents having 1 to 3 carbon atoms,
p31 and p32 each represent, independently of each other, 0 or 1, and
n³ represents an integer of equal to or greater than 1 corresponding to the number of the structure units parenthesized with [ ], provided that when n³ is equal to or greater than 2, then the two or more structure units in [ ] may be either identical to each other or different from each other. 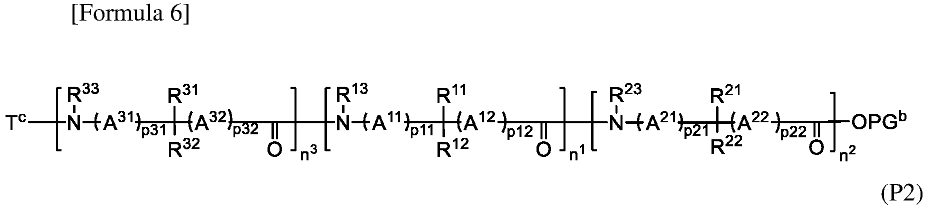
In formula (P2),
R¹¹, R¹², R¹³, A¹¹, A¹², p11, p12, and n¹ each represent the same definitions as those of the same symbols in general formula (R1) above,
PG^{b}, R²¹, R²², R²³, A²¹, A²², p21, p22, and n² each represent the same definitions as those of the same symbols in general formula (R2) above, and
T^{c}, R³¹, R³², R³³, A³¹, A³², p31, p32, and n³ each represent the same definitions as those of the same symbols in general formula (R3) above.

3. The method according to claim 1 or 2, wherein a silane compound is added to the reaction system.

4. The method according to any one of claims 1 to 3, wherein the reaction is carried out as a batch reaction or a flow reaction.

5. An amidation reaction agent for use in the method according to any one of claims 1 to 4, comprising an aluminum compound represented by formula (A). 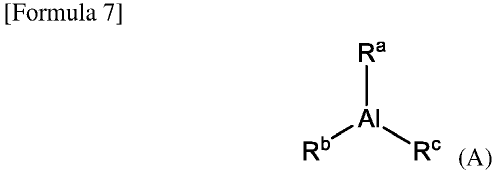
In formula (A),
R^{a}, R^{b}, and R^{c} each represent, independently of each other, a hydrogen atom, halogen atom, hydroxyl group, carboxyl group, nitro group, cyano group, or thiol group, or a monovalent aliphatic hydrocarbon group, aromatic hydrocarbon group, heterocyclic group, aliphatic hydrocarbon oxy group, aromatic hydrocarbon oxy group, or heterocyclic oxy group that may have one or more substituents.
